# EUROPEAN PATENT SPECIFICATION

(11) **EP 3 016 623 B1**
(45) Date of publication and mention of the grant of the patent: **06.08.2025**
(21) Application number: 14744475.6
(22) Date of filing: 30.06.2014
(51) Int. Cl.: A61F 13/20, A61F 13/26

(54) **TAMPON APPLICATOR AND METHOD FOR ITS ASSEMBLY**
TAMPONAPPLIKATOR UND VERFAHREN ZU DESSEN MONTAGE
APPLICATEUR DE TAMPON ET MÉTHODE D'ASSEMBLAGE

(30) Priority: 02.07.2013 EP 13174644
(43) Date of publication of application: 11.05.2016
(73) Proprietor: Ruggli AG, 5322 Koblenz (CH)
(72) Inventor: ROLLI, Kilian, CH-5400 Baden (CH); HAMMEN, Axel, CH-5426 Lengnau (CH)
(74) Representative: IPrime Rentsch Kaelin AG
(86) International application number: PCT/EP2014/063807
(87) International publication number: WO 2015/000827

(56) References cited:
- GB-A- 549 053
- US-A- 2 854 978
- US-A- 3 148 680
- US-A- 4 573 963
- US-A- 4 573 964
- US-A- 5 453 085
- US-A1- 2002 010 413
- US-A1- 2010 324 468
- US-B1- 6 248 089
- US-B1- 6 432 076

## Description

The invention relates to a tampon applicator comprising a barrel for receiving a tampon and a plunger for ejecting the tampon from the barrel, the barrel having an insertion end and a plunger end and in the area of its plunger end a guide section for the plunger, the plunger having a first enlarged end for contact with the tampon and a second enlarged end for contact with a finger, and the internal diameter of the guide section of the barrel being smaller than the external diameter of the first and second ends of the plunger.

Tampon applicators of this kind are known in the art. In these applicators, the enlarged ends of the plungers not only provide an enlarged contact area for the tampon and the finger, respectively, but they also prevent that the plunger may fall out of the barrel. Up to now, two methods for introducing the plunger into the barrel have become known. According to patent US7044928B2, the plunger is first manufactured as a hollow cylinder and introduced into the guide section of the barrel, and the ends of the plunger are then widened by means of a heated tool. According to patent application WO2012107880A2, the guide section is formed by a plurality of ridges extending radially inwards in the plunger end of the barrel, and an enlarged end of the plunger is pushed into the guide section such that the enlarged portion is deformed during the movement through the guide section and restored after leaving the guide section.

It is obvious that the method according to US7044928B2 is relatively laborious as the initially cylindrical plunger has to be introduced into the barrel and then widened on both sides. The method according to WO201210788042 is only applicable with hollow, relatively thin-walled plungers since one of the enlarged ends of the plunger has to be deformable by the radial ridges in the guide section. Plungers that are not hollow and relatively thick-walled plungers, as well as plungers where the end that is to be pushed through the guide section has an increased wall thickness, cannot be mounted with this method. An increase in wall thickness may e.g. be the result of the thermal deformation of a cylindrical plunger end or of the manufacture of the plunger by injection molding.

**The documents** US3148680**,** US2854978 **and** US4573964 **show similar tampon applicators with guided plungers.**

On the background of this prior art it is the object of the invention to suggest a tampon applicator in which the plunger is easy to mount even if it is thick-walled or at least has an end having an increased wall thickness.

According to the invention, this object is achieved in that the guide section is formed by a number of radially resilient elastic fins as defined by claim 1.

In particular, this solution according to the invention offers the advantage that the elastic fins yield elastically while the plunger is being mounted and therefore no specific requirements apply to the plunger as to its elasticity. Moreover, since the elastic fins are radially resilient, they may be dimensioned so as to lie against the portion between the ends of the mounted plunger without play and thus optimally guide the plunger. Furthermore, the elastic fins are able to guide the plunger on a large part of its circumference, which is not possible with the ridges according to WO2012107880A2.

The terms "internal diameter" and "external diameter" as used in the present context are not meant to limit the invention to barrels and plungers of circular cross-section but shall also include other cross-sectional shapes such as oval or polygonal ones.

According to one embodiment of the invention, the barrel has a receiving section for the tampon and a gripping section arranged in the area of its plunger end and whose external diameter is smaller than the external diameter of the receiving section. This allows a better handling of the tampon applicator.

According to a further embodiment, the plunger is hollow and the first end and/or the second end of the plunger has a greater wall thickness than the portion of the plunger between its ends. This configuration simplifies the manufacture of the plunger in that the plunger may e.g. be produced by injection-molding.

According to the invention, the elastic fins extend from the inner circumference of the barrel in the longitudinal direction of the barrel and are inclined toward the longitudinal axis of the barrel. This inclination simplifies the insertion of the preceding plunger end while the plunger is being mounted.

According to the invention, the elastic fins have respective first sections extending, as seen in cross-section, from the inner circumference of the barrel in the direction of a chord, followed by second sections extending substantially parallelly to the inner circumference of the barrel. The area in which the elastic fins are connected to the wall of the barrel extends in the longitudinal direction of the barrel substantially.

According to a further embodiment, the elastic fins are bent more than 90 degrees between their first sections and their second sections. The bent sections act like cantilever springs, and allow a shorter design of the elastic fins in the circumferential direction, which in turn allows arranging a greater number of elastic fins.

According to a further embodiment, the second sections of the elastic fins extend on both sides of their first sections in the circumferential direction. This allows a nearly gapless conformation of the guide section.

Another aspect of the invention relates to the assembly of the tampon applicator of the invention, as defined by claim 6, which should also be possible with a relatively rigid plunger, in the extreme case even with a plunger that is not hollow. Accordingly, it is suggested that the plunger is mounted by being pushed through the guide section from one end of the barrel, that the preceding plunger end forces the elastic fins outwards in a substantially radial direction so that the diameter of the guide section is temporarily enlarged, and that after the passage of the preceding end through the guide section, the elastic fins return to their positions prior to the passage of the preceding plunger end.

Exemplary embodiments of the invention are shown in figures 5 to 7, and will be described in more detail hereinafter with reference to the appended drawings showing
- Figure 1: a longitudinal section of a tampon applicator assembly;
- Figure 2: a detail of Figure 1 on an enlarged scale relative to Figure 1, without the tampon and the withdrawal string;
- Figure 3: a perspective view of the barrel according to Figures 1 and 2;
- Figure 4: a detail of Figure 1 on an enlarged scale relative to Figure 1 without the tampon and the withdrawal string, which shows the finger enlargement of the plunger;
- Figure 5: a view of an embodiment of the barrel of the tampon applicator according to the invention, as seen from the plunger end;
- Figure 6: a view of an embodiment of the barrel of the tampon applicator according to the invention, as seen from the plunger end; and
- Figure 7: a view of an embodiment of the barrel of the tampon applicator according to the invention, as seen from the plunger end.

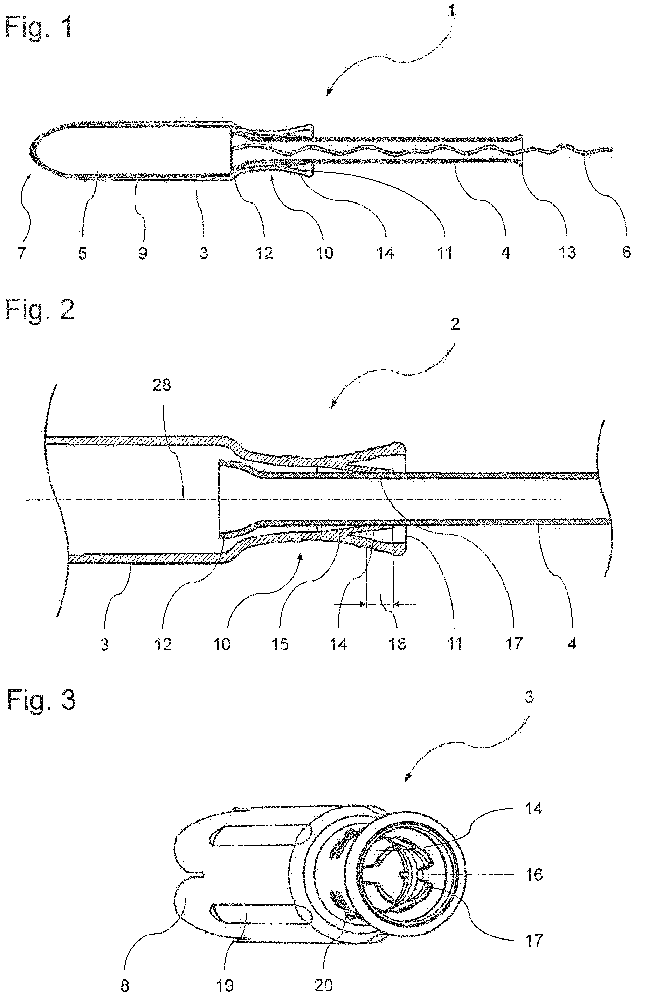

Figures 1 to 4 show a tampon applicator 2, not according to the invention, Figure 1 showing an overview of a tampon applicator assembly 1 in a longitudinal section. Assembly 1 consists of a barrel 3 having a longitudinal axis 28, a tampon 5 received in a receiving section 9 of barrel 3 and having a withdrawal string 6, and a plunger 4 for ejecting tampon 5 from barrel 3. Barrel 3 and plunger 4 form the tampon applicator. In the illustration of Figure 1, the insertion end 7 of assembly 1 is closed by a number of lips 8 (see Figure 3) that are integrally formed on barrel 3 and are bent inwards. In Figure 3, which shows barrel 3 alone in a perspective view, lips 8 are illustrated in the open position. At its plunger end 11, barrel 3 has a gripping section 10 having a reduced diameter relative to receiving section 9. This diameter reduction is not compulsory; gripping section 10 might alternatively have the same external diameter as receiving section 9. At its ends, plunger 4 has a tampon side enlargement 12 and a finger enlargement 13.

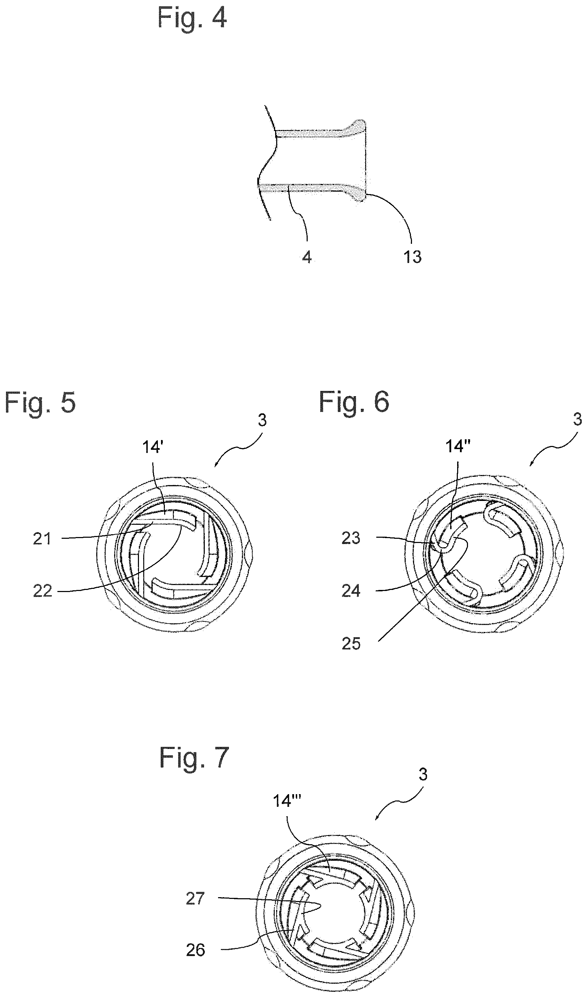

Figure 2 shows a detail of Figure 1, however without tampon 5 and withdrawal string 6 and on an enlarged scale relative to Figure 1. On the inner circumference of gripping section 10, elastic fins 14 are arranged that are connected by their foot sections 15 to the wall of barrel 3 and whose free ends are oriented inwards and in the direction of the plunger end of barrel 3. The contact surfaces 17 of elastic fins 14 on plunger 4 form a guide section 18 for plunger 4. In the depicted example, four elastic fins 14 are arranged on the inner circumference of gripping section 10, while it is seen in Figure 3 that between two respective elastic fins 14 a gap 16 is provided such that the free ends of elastic fins 14 may elastically move outwards and inwards radially. In receiving section 9 of barrel 3, inwardly or outwardly projecting ridges 19 may be provided for increased stiffness, and in gripping section 10, a gripping structure 20 may be arranged to prevent slipping of the fingers during the insertion of assembly 1. Figure 4 shows a detail of plunger 4 with finger enlargement 13 and clearly illustrates that this area may have an increased wall thickness since this portion - as opposed to the prior art known from reference WO2012107880A2 - is not subject to a deformation during the assembly of the plunger.

To mount plunger 4 in barrel 3, plunger 4 is introduced into insertion end 7 of barrel 3 with finger enlargement 13 first. During the passage of finger enlargement 13 through gripping section 10, the free ends of elastic fins 14 are forced radially outwards by finger enlargement 13, resiliently return after the passage of finger enlargement 13, and then lie against the outer circumference of middle section of plunger 4.

Alternatively, which is not illustrated in the drawings, the free ends of elastic fins 14 may be directed against insertion end 7 of barrel 3. In this case, the plunger is mounted from plunger end 11 of barrel 3, plunger 4 being introduced into the interior of gripping section 10 with tampon side enlargement 12 first.

Figures 5 to 7 show embodiments of a barrel 3 of tampon applicator 2 according to the invention as seen from plunger end 11. In the embodiment according to Figure 5, elastic fins 14' are composed of respective straight sections 21 extending from the wall of barrel 3 in the direction of a chord and of adjoining curved contact sections 22 for the plunger. The indication "direction of a chord" means that the corresponding area is not directed towards the cross-sectional center of barrel 3 but approximately tangential to plunger 4 received in guide section 18. In this manner, elastic fins 14', 14'', and 14‴ are capable of resiliently yielding radially outwards during the passage of finger enlargement 13 while the plunger is being mounted. In the embodiment according to Figure 6, elastic fins 14" also have straight sections 23 which also project from the wall of barrel 3 in the direction of a chord but are shorter than the straight sections 21 in the exemplary embodiment according to Figure 5. These straight sections 23 are followed by arcs 24 that are in turn followed by contact sections 25 for the plunger. Due to this configuration, elastic fins 14'' require less space in the circumferential direction than elastic fins 14' according to the previously described example. In this manner, e.g. five or six elastic fins 14'' can be arranged on the inner circumference of the gripping section. In the exemplary embodiment according to Figure 7 also, straight sections 26 extending from the wall of barrel 3 in the direction of a chord are again provided. At their ends, they carry contact sections 27 which project on both sides from the straight sections 26 in the circumferential direction and thus ensure a particularly effective guidance of plunger 4.

In the exemplary embodiments of Figures 5 to 7, elastic fins 14', 14'', and 14‴ are arranged such that their sections that are to be contacted by one end of plunger 4 while plunger 4 is being mounted have a larger clear diameter at the location where they are first contacted during the insertion of plunger 4. In the illustrated examples, the clear diameter defined by contact sections 22, 25, and 27 conically decreases towards the plunger end 11 of barrel 3. Accordingly, in these exemplary embodiments, the plunger is mounted from the insertion end 7 of barrel 3 with finger enlargement 13 first. In alternative examples that are not illustrated in the drawings, the inclination of the aforementioned sections might be inverse so that plunger 4 could be mounted from the plunger end 11 of barrel 3 with tampon side enlargement 12 first.

In the exemplary embodiments of Figures 5 to 7, contact sections 22, 25, and 27 are not only connected to the wall of barrel 3 by straight sections 21, 23, and 26, but additionally on their sides facing away from the viewer, similarly to the foot sections 15 in the embodiment according to Figures 1 to 3. This is not compulsory, however, but each contact section 22, 25, and 27 may have two free ends in the longitudinal direction of barrel 3, namely as illustrated in the area of plunger end 11 and as opposed to the illustrations on their sides facing away from plunger end 11.

In all illustrated and previously described exemplary embodiments, four elastic fins 14', 14'', or 14‴ are distributed on the inner circumference of gripping section 10. However, the invention is not limited to four elastic fins but two, three, five, six, or more elastic fins may be provided.

In all illustrated and previously described exemplary embodiments, barrel 3 is preferably made of a preferentially thermoplastic material and is preferably produced by the injection molding technique. Elastic fins 14', 14", or 14‴ are preferably formed integrally with barrel 3. Alternatively, however, elastic fins 14', 14'', or 14‴ may e.g. be formed on a ring (not shown) that is connected to the barrel, e.g. by a snap connection. In this manner, the range of possible designs of the elastic fins is extended with regard to tool techniques in that elastic fins may be manufactured that would be difficult or impossible to realize integrally with the barrel.

Ultimately, as a matter of form, it should be noted that for a better understanding of the structure of the tampon applicator assembly, the latter or its components, respectively, are partly illustrated out of scale and/or on an enlarged or reduced scale.

### List of Reference Numerals

| | | | |
|---|---|---|---|
| 1 | tampon applicator assembly | 21 | straight section |
| | | 22 | contact section |
| 2 | tampon applicator | 23 | straight section |
| 3 | barrel | 24 | arc |
| 4 | plunger | 25 | contact section |
| 5 | tampon | 26 | straight section |
| 6 | withdrawal string | 27 | contact section |
| 7 | insertion end | 28 | longitudinal axis |
| 8 | lips | | |
| 9 | receiving section | | |
| 10 | gripping section | | |
| 11 | plunger end | | |
| 12 | tampon side enlargement | | |
| 13 | finger enlargement | | |
| 14 | elastic fin | | |
| 14' | elastic fin | | |
| 14" | elastic fin | | |
| 14‴ | elastic fin | | |
| 15 | foot section | | |
| 16 | gap | | |
| 17 | contact surface | | |
| 18 | guide section | | |
| 19 | ridge | | |
| 20 | gripping structure | | |

## Claims

1. Tampon applicator (2) comprising a barrel (3) for receiving a tampon (5) and a plunger (4) for ejecting the tampon (5) from the barrel (3), the barrel (3) having an insertion end (7) and a plunger end (11) and in the area of its plunger end a guide section (18) for the plunger (4), the plunger (4) having a first enlarged end (12) for contact with the tampon (5) and a second enlarged end (13) for contact with a finger, and the internal diameter of the guide section (18) of the barrel (3) being smaller than the external diameter of the first and second ends (12, 13) of the plunger (4), the guide section (18) being formed by a number of radially resilient elastic fins ( 14'; 14"; 14‴) that are distributed on the inner circumference of the barrel (3), wherein the elastic fins (14) extend from the inner circumference of the barrel (3) in the longitudinal direction of the barrel and are inclined toward the longitudinal axis (28) of the barrel (3), and **characterized in that** the elastic fins (14'; 14"; 14‴) have respective first sections (21; 23; 26) extending, as seen in cross-section, from the inner circumference of the barrel (3) in the direction of a chord, followed by second sections (22; 25; 27) extending substantially parallelly to the inner circumference of the barrel (3).

2. Tampon applicator (2) according to claim 1, **characterized in that** the barrel (3) has a receiving section (9) for the tampon (5) and a gripping section (10) arranged in the area of its plunger end (11) and whose external diameter is smaller than the external diameter of the receiving section (9).

3. Tampon applicator (2) according to one of the preceding claims, **characterized in that** the plunger (4) is hollow and that the first end (12) and/or the second end (13) of the plunger (4) has a greater wall thickness than the portion of the plunger (4) between its ends (12, 13).

4. Tampon applicator according to claim 1, **characterized in that** the elastic fins (14") are bent more than 90 degrees between their first sections (23) and their second sections (25).

5. Tampon applicator according to claim1, **characterized in that** the second sections (27) of the elastic fins (14'") extend on both sides of their first sections (26) in the circumferential direction.

6. Method for the assembly of the tampon applicator (2) according to one of the preceding claims, **characterized in that** the plunger (4) is mounted by being pushed through the guide section (18) from one end (7, 11) of the barrel (3), that the preceding end (12, 13) of the plunger (4) thereby forces the elastic fins (14; 14'; 14"; 14'") outwards in a substantially radial direction so that the diameter of the guide section (18) is temporarily enlarged, and that after the passage of the preceding end (12, 13) through the guide section (18), the elastic fins ( 14'; 14"; 14‴) return to their positions prior to the passage of the preceding end (12, 13) of the plunger (4).

## Patentansprüche

1. Tamponapplikator (2), umfassend eine Hülse (3) zur Aufnahme eines Tampons (5) und einen Stößel (4) zum Ausstoßen des Tampons (5) aus der Hülse (3), wobei die Hülse (3) ein Einführende (7) und ein Stößelende (11) und im Bereich seines Stößelendes einen Führungsabschnitt (18) für den Stößel (4) aufweist, wobei der Stößel (4) ein erstes vergrößertes Ende (12) für den Kontakt mit dem Tampon (5) und ein zweites vergrößertes Ende (13) für den Kontakt mit einem Finger aufweist und der Innendurchmesser des Führungsabschnitts (18) der Hülse (3) kleiner ist als der Außendurchmesser des ersten und des zweiten Endes (12, 13) des Stößels (4), wobei der Führungsabschnitt (18) durch eine am Innenumfang der Hülse (3) verteilte Anzahl radial nachgiebiger elastischer Lamellen (14'; 14"; 14‴) gebildet ist, wobei die elastischen Lamellen (14) sich vom Innenumfang der Hülse (3) in Längsrichtung der Hülse erstrecken und zur Längsachse (28) der Hülse (3) hin geneigt sind, und **dadurch gekennzeichnet, dass** die elastischen Lamellen (14'; 14"; 14‴) jeweilige erste Abschnitte (21; 23; 26) aufweisen, die sich im Querschnitt gesehen vom Innenumfang der Hülse (3) in Richtung einer Sehne erstrecken, gefolgt von zweiten Abschnitten (22; 25; 27), die sich im Wesentlichen parallel zum Innenumfang der Hülse (3) erstrecken.

2. Tamponapplikator (2) nach Anspruch 1, **dadurch gekennzeichnet, dass** die Hülse (3) einen Aufnahmeabschnitt (9) für den Tampon (5) und einen im Bereich seines Stößelendes (11) angeordneten Griffabschnitt (10) aufweist, dessen Außendurchmesser kleiner ist als der Außendurchmesser des Aufnahmeabschnitts (9).

3. Tamponapplikator (2) nach einem der vorhergehenden Ansprüche, **dadurch gekennzeichnet, dass** der Stößel (4) hohl ist und dass das erste Ende (12) und/oder das zweite Ende (13) des Stößels (4) eine größere Wandstärke aufweist als der Abschnitt des Stößels (4) zwischen seinen Enden (12, 13).

4. Tamponapplikator nach Anspruch 1, **dadurch gekennzeichnet, dass** die elastischen Lamellen (14") zwischen ihren ersten Abschnitten (23) und ihren zweiten Abschnitten (25) um mehr als 90 Grad gebogen sind.

5. Tamponapplikator nach Anspruch 1, **dadurch gekennzeichnet, dass** sich die zweiten Abschnitte (27) der elastischen Lamellen (14‴) auf beiden Seiten ihrer ersten Abschnitte (26) in Umfangsrichtung erstrecken.

6. Verfahren zur Montage des Tamponapplikators (2) nach einem der vorhergehenden Ansprüche, **dadurch gekennzeichnet, dass** der Stößel (4) montiert wird, indem er von einem Ende (7, 11) der Hülse (3) durch den Führungsabschnitt (18) geschoben wird, dass dabei das vorangehende Ende (12, 13) des Stößels (4) die elastischen Lamellen (14; 14'; 14"; 14‴) in einer im Wesentlichen radialen Richtung nach außen drängt, so dass der Durchmesser des Führungsabschnitts (18) vorübergehend vergrößert wird, und dass nach dem Durchgang des vorangehenden Endes (12, 13) durch den Führungsabschnitt (18) die elastischen Lamellen (14'; 14"; 14‴) in ihre Positionen vor dem Durchgang des vorangehenden Endes (12, 13) des Stößels (4) zurückkehren.

## Revendications

1. Applicateur de tampon (2) comprenant un cylindre (3) pour recevoir un tampon (5) et un poussoir (4) pour éjecter le tampon (5) hors du cylindre (3), le cylindre (3) ayant une extrémité d'insertion (7) et une extrémité de poussoir (11) et, dans la zone de son extrémité de poussoir, une section de guidage (18) pour le poussoir (4), le poussoir (4) ayant une première extrémité élargie (12) pour venir en contact avec le tampon (5) et une deuxième extrémité élargie (13) pour venir en contact avec un doigt, et le diamètre intérieur de la section de guidage (18) du cylindre (3) étant inférieur au diamètre extérieur des première et deuxième extrémités (12, 13) du poussoir (4), la section de guidage (18) étant formée par un nombre d'ailettes élastiques radialement souples (14'; 14" ; 14"') qui sont réparties sur la circonférence intérieure du cylindre (3), les ailettes élastiques (14) s'étendant depuis la circonférence intérieure du cylindre (3) dans la direction longitudinale du cylindre et étant inclinées vers l'axe longitudinal (28) du cylindre (3), et **caractérisé en ce que** les ailettes élastiques (14'; 14" ; 14"') ont des premières sections respectives (21; 23 ; 26) s'étendant, telles que vues en coupe transversale, depuis la circonférence intérieure du cylindre (3) dans la direction d'une corde, suivies de deuxièmes sections (22 ; 25 ; 27) s'étendant essentiellement parallèlement à la circonférence intérieure du cylindre (3).

2. Applicateur de tampon (2) selon la revendication 1, **caractérisé en ce que** le cylindre (3) a une section de réception (9) pour le tampon (5) et une section de préhension (10) agencée dans la zone de son extrémité de poussoir (11) et dont le diamètre extérieur est inférieur au diamètre extérieur de la section de réception (9).

3. Applicateur de tampon (2) selon l'une quelconque des revendications précédentes, **caractérisé en ce que** le poussoir (4) est creux et **en ce que** la première extrémité (12) et/ou la deuxième extrémité (13) du poussoir (4) a une épaisseur de paroi supérieure à celle de la partie du poussoir (4) entre ses extrémités (12, 13).

4. Applicateur de tampon selon la revendication 1, **caractérisé en ce que** les ailettes élastiques (14") sont pliées à plus de 90 degrés entre leurs premières sections (23) et leurs deuxièmes sections (25).

5. Applicateur de tampon selon la revendication 1, **caractérisé en ce que** les deuxièmes sections (27) des ailettes élastiques (14") s'étendent des deux côtés de leurs premières sections (26) dans la direction circonférentielle.

6. Procédé d'assemblage de l'applicateur de tampon (2) selon l'une quelconque des revendications précédentes, **caractérisé en ce que** le poussoir (4) est monté en étant poussé à travers la section de guidage (18) depuis une extrémité (7, 11) du cylindre (3), **en ce que** l'extrémité précédente (12, 13) du poussoir (4) force ainsi les ailettes élastiques (14 ; 14' ; 14"; 14"') vers l'extérieur dans une direction essentiellement radiale, de telle sorte que le diamètre de la section de guidage (18) est temporairement élargi, et **en ce qu'**après le passage de l'extrémité précédente (12, 13) à travers la section de guidage (18), les ailettes élastiques (14' ; 14" ; 14"') reviennent à leurs positions avant le passage de l'extrémité précédente (12, 13) du poussoir (4).
